# FASCICULE DE BREVET EUROPEEN

(11) **EP 1 513 735 B1**
(45) Date de publication et mention de la délivrance du brevet: **19.03.2008**
(21) Numéro de dépôt: 03752805.6
(22) Date de dépôt: 14.05.2003
(51) Int. Cl.: B65D 47/42, B65D 35/36, A61M 35/00

(54) **EMBALLAGE APPLICATEUR DE COMPOSITION COULANTE**
VERPACKUNG ZUM AUFBRINGEN EINER FLIESSFÄHIGEN ZUSAMMENSETZUNG
FLOWABLE COMPOSITION APPLYING PACKAGE

(30) Priorité: 22.05.2002 FR 0206222
(43) Date de publication de la demande: 16.03.2005
(73) Titulaire: J.S.O., 82350 Albias (FR)
(72) Inventeur: SAUNIERE, JEAN, 40150 Hossegor (FR); OLMI, Eugène, 77650 Sainte-Colombe (FR); COHEN, Edouard, 92200 Neuilly-sur-Seine (FR)
(74) Mandataire: Cabinet BARRE LAFORGUE & associés
(86) Numéro de dépôt international: PCT/FR2003/001463
(87) Numéro de publication internationale: WO 2003/097476

(56) Documents cités:
- DE-U- 20 011 706
- FR-A- 2 422 564
- FR-A- 2 795 928
- GB-A- 433 601
- GB-A- 751 237

## Description

L'invention concerne un emballage applicateur d'une composition coulante (liquide, semi-liquide, pâteuse, voire éventuellement pulvérulente), du type comprenant un réservoir de composition coulante et un tampon applicateur. L'invention concerne en particulier un emballage applicateur de composition coulante topique médicale, pharmaceutique, thérapeutique, dermatologique ou cosmétique à usage externe, destinée à être appliquée sur la peau par exemple, ou encore de composition coulante nettoyante, détergente (savon dit savon liquide par exemple), nourrissante (cirage pour chaussures par exemple), de peinture, etc., destinée à être appliquée sur une surface à traiter quelconque.

Les emballages connus à tampon applicateur de composition coulante sont de deux types.

Un premier type regroupe les emballages applicateurs à tampon poreux perméable à la composition coulante emballée.

Pour éviter l'écoulement intempestif de la composition coulante en dehors de toute utilisation de l'applicateur, une première approche consiste à prévoir des moyens mécaniques de rétention de la composition dans la réservoir.

On connaît ainsi des moyens mécaniques de rétention montés de manière amovible à l'extérieur du tampon pour en obturer la face d'application en vue d'empêcher l'écoulement et/ou l'évaporation de la composition liquide. Tel est le cas du capuchon décrit par FR-2.800.041, qui vient se visser sur le col du réservoir à l'intérieur duquel est logé le tampon. L'inconvénient de ces emballages applicateurs est qu'ils présentent un tampon applicateur constamment imbibé et saturé de composition coulante, et ne permettent donc pas à l'utilisateur d'agir sur le débit de diffusion et de libération de ladite composition en vue d'en contrôler l'application.

On connaît aussi des moyens mécaniques de rétention de la composition coulante interposés à l'intérieur de l'emballage applicateur, entre le réservoir et le tampon applicateur, en vue d'empêcher l'imprégnation du tampon par la composition coulante en dehors de toute utilisation de l'applicateur. Ainsi, DE 200 11 706 U décrit un dispositif d'application incorporant une feuille de séparation entre le réservoir et le tampon applicateur, et des "moyens d'activation" qui, lorsqu'on exerce une pression sur le réservoir ou sur une feuille de protection amovible recouvrant le tampon applicateur, viennent déchirer la feuille de séparation. WO 02/08082 décrit un applicateur comprenant un réservoir de liquide flexible, un dôme poreux monté sur une ouverture du réservoir de façon à définir une chambre intermédiaire entre le réservoir et le dôme, un tube d'alimentation de ladite chambre en liquide s'étendant entre le réservoir et la chambre. L'augmentation de la pression dans le réservoir flexible, par pincement manuel de celui-ci par l'utilisateur, entraîne le déplacement d'une dose de liquide depuis le réservoir jusqu'à la chambre par le tube d'alimentation, et l'imprégnation subséquente du tampon poreux. Le relâchement du réservoir fait chuter la pression à l'intérieur de celui-ci et entraîne l'aspiration de l'excès de liquide restant dans la chambre. L'applicateur comprend de plus un capuchon extérieur de protection du dôme poreux, et une vanne agencée dans le tube d'alimentation, automatiquement fermée lorsque le capuchon est vissé sur le réservoir. Un autre exemple de moyens mécaniques interposés entre le réservoir et le tampon, comprenant un mécanisme (doté d'une valve à bille) pour le pompage d'une dose de fluide depuis le réservoir vers la tête d'application, est décrit par WO 90/15567. Ces emballages applicateurs sont techniquement complexes, comprennent un grand nombre de pièces, et sont particulièrement coûteux. A noter que, pour ces raisons, ils ne peuvent pas être utilisés pour emballer une dose unitaire (pour un usage unique) de composition coulante.

Pour éviter une libération intempestive de composition coulante avec les emballages de premier type, une deuxième approche connue consiste à traiter la composition coulante préalablement à son emballage en vue de la transformer en un produit susceptible d'être retenu par le tampon applicateur. Ainsi, FR-2.739.003, qui décrit une houppette de mousse constituée de deux plaques de mousse superposées, prévoit une étape préalable de réduction de la composition liquide à emballer en une poudre (ou éventuellement un gel solide) hydrosoluble ou émulsifiable, par lyophilisation, atomisation, déshydratation, ou cristallisation de ladite composition. Lorsque l'on humidifie la houppette, la poudre se dissout ou s'émulsionne, et la solution liquide ou pâteuse obtenue s'écoule à travers l'une des plaques de mousse pour être appliquée par tapotement sur la surface à traiter. Les inventeurs ont constaté que la dissolution ou l'émulsion de la poudre entre les deux plaques de mousse ne conduisait pas à l'obtention d'une solution parfaitement homogène, de sorte qu'il est difficile d'appliquer uniformément sur la surface à traiter une composition ainsi emballée. Le choix d'un tel emballage suppose, de plus, que la composition coulante à emballer soit réductible en poudre et apte à conserver ses propriétés après avoir subi une telle réduction, et soit également soluble ou émulsifiable dans l'eau et apte à retrouver ses propriétés suite à cette nouvelle transformation. D'autres procédés d'emballage connus enseignent d'encapsuler la composition coulante préalablement à son introduction dans le réservoir de l'emballage applicateur. L'humidification d'un tel emballage applicateur entraîne la dissolution des capsules enfermant la composition coulante et la libération subséquente de celle-ci. Dans les deux cas précédents (composition déshydratée ou encapsulée), l'application de la composition ne peut se faire sans eau. De plus, la solution obtenue diffuse immédiatement à travers la mousse du tampon et sa libération ne peut être quantitativement contrôlée.

Le second type d'emballages applicateurs regroupe les emballages applicateurs à tampon imperméable à la composition coulante emballée.

Ces emballages applicateurs présentent alors nécessairement des conduits d'évacuation de la composition coulante traversant le tampon pour permettre de véhiculer la composition coulante depuis le réservoir jusqu'à la face d'application dudit tampon. Ces conduits sont usuellement fermés au moyen de valves, ou d'autres moyens adaptés, en dehors de toute utilisation de l'applicateur. Ainsi, US-5.871.020 décrit un applicateur comprenant un réservoir flexible contenant une préparation liquide ou visqueuse, et une tête d'application, laquelle tête comprend une paroi transversale séparant le réservoir d'une mousse d'application à cellules fermées dans laquelle ne pénètre pas la préparation. La paroi transversale est dotée d'au moins une ouverture, et la mousse présente au moins une fente s'étendant en regard de chaque ouverture entre sa face interne de fixation sur la paroi transversale et sa face externe d'application. En position de repos, chaque fente est fermée (parois de la fente pressées l'une contre l'autre). Une pression exercée sur le réservoir flexible permet d'écarter les parois de chaque fente pour laisser couler la préparation liquide ou visqueuse le long de ladite fente. Le dispositif comprend également un capuchon de fermeture permettant de compresser la mousse radialement et/ou verticalement en vue de garantir une fermeture étanche des fentes durant les périodes de non-utilisation de l'applicateur. La mousse des tampons applicateurs des emballages du second type n'étant pas perméable à la composition coulante, la quantité de composition coulante libérée reste concentrée autour des fentes ou conduits d'évacuation. Dès lors, lorsque le tampon est pressé contre la peau ou la surface à traiter, la composition n'est déposée qu'en certains points. Les dépôts ponctuels de composition sont ensuite étalés sur la zone de surface à traiter par massage de ladite zone au moyen du tampon, ce massage entraînant également l'étalement de la composition sur la face d'application du tampon. Il est, dans ces conditions, difficile d'obtenir une application sur la peau qui soit uniforme, et ce, d'autant plus si la composition coulante pénètre rapidement dans l'épiderme (ou la surface à traiter). Une concentration accrue de composition est alors absorbée en regard des fentes ou conduits au moment où le tampon est pressé contre la peau. Enfin, les emballages du second type, de par leur complexité (présence de conduits d'évacuation, valves, ...) présentent des coûts de fabrication élevés.

L'invention vise à pallier l'ensemble de ces inconvénients, et notamment à proposer un emballage applicateur de composition coulante qui soit simple, peu coûteux, puisse s'utiliser sans eau, laisse à l'utilisateur la possibilité d'agir sur le débit de libération de la composition coulante, et permette d'obtenir une application relativement uniforme de la composition sur la surface à traiter.

En particulier, l'invention vise à proposer un emballage applicateur doté d'un tampon applicateur qui soit au moins partiellement, et sous certaines conditions, perméable à la composition coulante, mais dont la face d'application au moins (et de préférence la quasi-totalité de l'épaisseur du tampon) reste exempte de composition coulante en dehors de toute utilisation de l'applicateur.

Un autre objectif de l'invention est de proposer un emballage applicateur qui permette de conditionner la composition coulante dans l'état dans lequel elle sera appliquée. L'invention vise notamment, à permettre d'emballer une composition coulante sans transformation ni traitement préalable (tel que déshydratation ou encapsulage) de ladite composition.

Un autre objectif de l'invention est de proposer un emballage applicateur qui soit, en tant qu'applicateur, ergonomique et facile d'utilisation.

L'invention vise également à proposer un emballage applicateur peu coûteux, et dont les coûts de fabrication soient compatibles avec l'offre d'un produit emballé contenant une dose unitaire de composition coulante.

Un autre objectif de l'invention est de proposer un emballage applicateur multi-réservoirs pour l'application simultanée d'une pluralité de compositions coulantes stockées séparément.

Pour ce faire, l'invention concerne un emballage applicateur de composition, dite composition coulante, conforme à la revendication 1.

Ainsi, la ou les matière(s) constitutive(s) du tampon applicateur elle(s)-même(s) est/sont adaptée(s) pour retenir la composition coulante dans le réservoir et empêcher l'imprégnation de la face d'application (et de la fraction d'épaisseur du tampon applicateur s'étendant entre la couche de filtrage et la face d'application) et l'écoulement spontané et intempestif de ladite composition en l'absence de pompage, et permettre une libération et une application de la composition coulante commandées sous l'effet du pompage.

Par "composition coulante", on entend une substance apte à s'écouler et susceptible de pouvoir subir un pompage par déplacement positif. Il peut s'agir d'un liquide, d'un produit semi-liquide, d'un gel; d'une crème, d'une pâté (en tout état de cause, d'un fluide incompressible ou éventuellement faiblement compressible), d'une substance hétérogène composée par exemple d'une poudre et d'un liquide, voire d'une poudre elle-même pour autant qu'elle conserve des propriétés d'écoulement et puisse être refoulée vers et à travers le tampon applicateur au moyen de là poire de pompage, une fois emballée selon l'invention.

Une "poire de pompage formant réservoir" désigne un réceptacle souple, flexible, élastique ou non, de forme quelconque adaptée pour permettre sa préhension et son pincement manuel, et dont on peut faire varier le volume par pincement en vue de refouler une composition coulante par déplacement positif. Au repos, une telle poire de pompage présente des forme et volume nominaux, qu'elle retrouve de préférence après pincement (cas d'une poire élastique). La poire de pompage selon l'invention est, par exemple évasée vers le tampon, notamment au moins sensiblement piriforme, pyramidale, tronconique, en pointe d'ellipsoïde, hémisphérique ou oblongue.

Un tampon applicateur est une plaque ou un bloc souple d'un seul tenant, exempt(e) de mécanisme, et constitué(e) essentiellement d'une pièce en matière(s) poreuse(s) à pores ouverts (les pores forment des interstices communiquant entre eux). La matière constitutive de chaque pièce peut être homogène ou composite.

La face interne du tampon applicateur selon l'invention est adaptée pour combler l'ouverture de distribution de la poire de pompage de façon à venir au contact direct de la composition coulante contenue dans le réservoir. Par "contact direct", on entend que l'emballage est dépourvu de toute paroi et/ou de tout moyen mécanique (conduit d'amenée avec valve, ...) interposé(e)(s) entre le réservoir et le tampon applicateur en vue de retenir la composition coulante dans le réservoir en dehors de toute utilisation de l'applicateur ; la composition coulante contenue dans le réservoir reste en permanence au contact du tampon (sensiblement sur toute la surface de celui-ci), et la fonction de rétention de la composition coulante est assurée par le tampon applicateur lui-même (ou au moins une partie dudit tampon dite couche de filtrage).

Le tampon applicateur selon l'invention comprend une couche de filtrage imperméable à la composition coulante en l'absence de pompage, et perméable à la composition coulante lorsque la poire de pompage est pincée. En l'absence de pompage, la diffusion de la composition coulante à travers le tampon applicateur est donc bloquée au niveau de la couche de filtrage. Selon l'invention, la couche de filtrage est continue, c'est-à-dire non perforée et non coupée, c'est-à-dire exempte de conduit ou fente traversant(e) ménagé(e) par découpage ou par perforation de ladite couche. L'expression "la couche de filtrage est en matière perméable à la composition coulante lorsque la poire de pompage est pincée" signifie que la matière constitutive de la couche de filtrage est adaptée pour autoriser la diffusion à travers elle (et non par des conduits ou fentes) de la composition coulante sous l'effet du pompage, mais n'exclut pas toutefois, dans le cas d'une matière composite, que celle-ci puisse incorporer des particules dispersées d'un matériau imperméable à la composition coulante (y compris lors du pompage).

Avantageusement et selon l'invention, la couche de filtrage s'étend sur une fraction au moins de l'épaisseur du tampon applicateur à partir de la face interne, en vue d'empêcher la pénétration de la composition coulante dans le tampon applicateur en l'absence de pincement de la poire pompage, ou de limiter cette pénétration à une très faible fraction de l'épaisseur du tampon applicateur. Le tampon reste ainsi exempt de composition coulante sur la quasi-totalité de son épaisseur en dehors de toute utilisation de l'emballage applicateur.

Les inventeurs ont montré que les propriétés de perméabilité et d'imperméabilité à la composition coulante de la matière constitutive de la couche de filtrage dépendent principalement de trois paramètres : la porosité (taille et nombre des pores) de ladite matière, la viscosité de la composition coulante, et la pression exercée manuellement sur la poire de pompage par l'utilisateur (au moment du pincement de celle-ci), intégralement transmise à la composition coulante lorsque le réservoir est plein (la composition coulante étant incompressible ou faiblement compressible). Les inventeurs ont également montré qu'il existe d'autres facteurs influençant ces propriétés -tels que les conditions d'utilisation de l'emballage applicateur (conditions de pression et de température atmosphériques), la nature physico-chimique de la matière constitutive de la couche de filtrage, sa tension de surface, son caractère hydrophobe ou hydrophile (pour les compositions coulantes aqueuses), la densité de la composition coulante et la taille des molécules qui la composent, etc.- mais que ceux-ci sont soit négligeables, soit variables dans une mesure trop faible pour être significative pour les applications courantes visées par l'invention, soit directement liés aux trois paramètres principaux susmentionnés.

Selon l'invention; la porosité de la couche de filtrage (c'est-à-dire de sa matière constitutive) est choisie en fonction de la viscosité de la composition coulante à appliquer de façon à permettre la rétention de la composition dans le réservoir en l'absence de pincement de la poire de pompage et sa libération contrôlée sous l'effet du pompage.

La pression de pompage exercée par l'utilisateur peut être évaluée à quelques centaines de grammes par centimètre carré, soit quelques Newton par centimètre carré (elle varie entre quelques dizaines de grammes et un kilogramme par cm² voire plus). La pression exercée par la composition coulante sur la face interne du tampon est par ailleurs sensiblement égale à cette pression de pompage.

Pour chaque composition coulante, la porosité de la couche de filtrage est donc choisie adaptée pour que ladite couche devienne perméable à la composition coulante lorsqu'une pression supérieure à une pression de pompage prédéterminée (de 200 grammes par centimètre carré par exemple) est exercée par l'utilisateur sur la poire de pompage. Cette porosité peut être exprimée en fonction du nombre de pores par unité de longueur selon une direction donnée, dit taux de porosité selon cette direction, qui est représentatif de la taille des pores selon ladite direction, et permet de quantifier les propriétés de perméabilité de la couche de filtrage selon cette direction (dans le cas avantageux d'une couche de filtrage en matière isotrope, le nombre de pores par unité de surface donne le nombre de pores par unité de volume et renseigne sur la perméabilité de ladite matière selon toutes les directions...). Dans toute la suite, les termes "taux de porosité" désignent le taux de porosité (de la couche de filtrage ou de toute autre partie du tampon applicateur) selon une direction au moins sensiblement orthogonale à la face d'application et/ou à la face interne du tampon applicateur, dite direction transversale.

Plus la composition coulante emballée est visqueuse, plus la taille des pores de la couche de filtrage (selon la direction transversale) devra être importante, et plus le taux de porosité sera choisi faible. Pour chaque composition coulante, on définit ainsi un taux de porosité seuil supérieur au-dessus duquel la couche de filtrage reste imperméable à la composition coulante en toute circonstance, y compris lorsque l'utilisateur exerce manuellement une pression sur la poire de pompage, ou bien au-dessus duquel il est nécessaire d'exercer sur la poire de pompage, en vue de libérer et appliquer la composition coulante, une pression importante significativement supérieure à celle normalement développée par un utilisateur lors du pompage, entre son pouce et son index (ou entre son pouce et son majeur) par exemple. A noter que les éventuelles autres parties du tampon applicateur présentent également, de préférence, un taux de porosité inférieur audit taux de porosité seuil supérieur pour permettre la diffusion à travers elles de la composition coulante sous l'effet du pompage (en variante, ces parties peuvent comprendre des conduits ou fentes). Dans une version préférée de l'invention, elles présentent un taux de porosité faible adapté pour permettre la diffusion naturelle (en l'absence de pompage) de ladite composition coulante.

Le taux de porosité de la couche de filtrage doit par ailleurs être supérieur à un taux seuil inférieur en dessous duquel la composition coulante peut diffuser dans la mousse en l'absence de toute contrainte exercée sur la poire de pompage et sous des conditions atmosphériques (pression, température, humidité ...) normales. De préférence, le taux de porosité de la couche de filtrage est supérieur à un taux seuil inférieur de sécurité en dessous duquel la composition coulante peut diffuser dans la mousse lorsque l'emballage applicateur est soumis à des conditions atmosphériques défavorables (température élevée, 30°C par exemple, susceptible de diminuer la viscosité de la composition coulante, et pression atmosphérique faible telle que celle rencontrée en altitude, 600 mbar par exemple) et qu'une faible contrainte, notamment significativement inférieure à celle normalement exercée par l'utilisateur lors du pompage, est appliquée sur la poire de pompage. Cette caractéristique permet d'éviter un écoulement intempestif et une libération incontrôlée de la composition coulante suite à des chocs légers éventuellement subis par l'emballage applicateur lors de son transport.

Pour chaque composition coulante, la couche de filtrage du tampon applicateur présente donc un taux de porosité compris entre les taux de porosité seuils inférieur et supérieur de la matière constitutive de ladite couche tels que définis ci-avant.

Avantageusement et selon l'invention, le tampon applicateur est constitué d'une seule pièce, c'est-à-dire d'une unique plaque (souple) ou d'un unique bloc (souple) en matière (homogène ou composite) poreuse perméable, dont une fraction au moins de l'épaisseur réalise la couche de filtrage.

Avantageusement et selon l'invention, le tampon applicateur est en mousse poreuse. Dans une version préférée de l'invention, le tampon applicateur est constitué d'une seule pièce en mousse poreuse. En variante, le tampon applicateur comprend une pluralité de pièces superposées ou juxtaposées, dont des pièces en mousse, papier, textile, plastique microperforé, etc.

Avantageusement et selon l'invention, la mousse du tampon applicateur présente, sur une fraction au moins de son épaisseur et en regard d'une portion au moins de l'ouverture de distribution, un taux de porosité compris entre 10 et 1000 ppp (pores par pouce, étant précisé que 1 pouce = 2,54 cm, et donc que 1 pore par pouce = 0,3937 pore par cm). Le taux de porosité est choisi de façon empirique, par tâtonnement successif, pour chaque composition coulante à emballer et pour une pression de pompage seuil prédéfinie qu'il est nécessaire d'exercer pour obtenir la libération de la composition coulante.

La porosité de la mousse du tampon applicateur peut être uniforme. Dans un tel mode de réalisation, l'intégralité du tampon constitue la couche de filtrage. Dans une variante avantageuse, la mousse du tampon applicateur présente une porosité variable selon la direction transversale.

En particulier, la mousse du tampon applicateur présente avantageusement, sur une fraction de son épaisseur à partir de la face interne (réalisant la couche de filtrage), un taux de porosité élevé, dit taux de porosité de filtrage, adapté pour empêcher la pénétration de la composition coulante dans le tampon applicateur en l'absence de pincement de la poire de pompage (le taux de porosité de filtrage est supérieur au taux de porosité seuil inférieur précédemment défini), et, sur une fraction de son épaisseur à partir de la face d'application, un taux de porosité plus faible, dit taux de porosité d'application, compatible avec la surface à traiter.

A titre d'exemple, dans le cas d'une composition coulante de viscosité dynamique comprise entre 0,5. 10⁻³ et 5 Pa.s (composition liquide, telle qu'une solution désinfectante -alcool à 90°, éosine, etc.-, une lotion démaquillante, un parfum, une huile...), la mousse du tampon applicateur présente un taux de porosité de filtrage compris entre 500 et 1000 pores par pouce (2,54cm) sur une épaisseur à partir de la face interne comprise entre 0,1 et 15 mm.

Dans le cas d'une composition coulante de viscosité dynamique comprise entre 5 et 15 Pa.s (produit plus visqueux, tel qu'un lait épais -lait démaquillant, lait solaire, etc.-, une crème de soin ou de beauté -crème teintée du type fond de teint, crème solaire, crème hydratante, crème à raser, etc.-, un gel -gel thérapeutique désinfectant, cicatrisant, antalgique, gel de massage, etc.-, une crème de cirage pour chaussures...), la mousse du tampon applicateur présente un taux de porosité de filtrage compris entre 60 et 500 pores par pouce (2,54cm) sur une épaisseur à partir de la face interne comprise entre 0,1 et 15 mm.

Dans le cas d'une composition coulante de viscosité dynamique comprise entre 15 et 1000 Pa.s (produit très visqueux, tel qu'une crème épaisse, une graisse, une résine polyester par exemple, ...), la mousse du tampon applicateur présente un taux de porosité de filtrage compris entre 10 et 60 pores par pouce (2,54cm) sur une épaisseur à partir de la face interne comprise entre 0,1 et 15 mm.

L'invention permet une libération contrôlée de la composition coulante, qui varie selon le taux de porosité de filtrage choisi et la pression exercée par l'utilisateur sur la poire de pompage. Le taux de porosité de filtrage peut être choisi en fonction de la destination du produit emballé (enfant, adulte...), des conditions de transport, de stockage (dans un sac à main par exemple) et d'utilisation dudit produit emballé, et en fonction du degré de contrôle souhaité. Un taux de porosité de filtrage faible se traduit, en effet, par une libération rapide et massive de la composition coulante sous l'effet d'une faible pression de l'utilisateur, alors qu'un taux de porosité de filtrage plus élevé autorise un contrôle plus fin de la distribution de la composition coulante.

Par ailleurs, dans le cas d'un emballage applicateur d'une composition cosmétique et/ou dermatologique (destinée à être appliquée sur la peau), le taux de porosité d'application est notamment choisi adapté pour rendre la face d'application agréable au toucher. Le taux de porosité d'application est de préférence également adapté pour permettre la diffusion naturelle de la composition coulante (il est donc inférieur au taux de porosité seuil inférieur précédemment défini), en vue, notamment, d'obtenir une répartition sensiblement uniforme de ladite composition sur la face d'application (lorsque la poire de pompage est pincée, et que la composition coulante pénètre dans l'épaisseur du tampon applicateur pour diffuser jusqu'à la face d'application). A titre d'exemple, la mousse du tampon applicateur présente avantageusement un taux de porosité d'application compris entre 10 et 200 pores par pouce (2,54cm), et de préférence compris entre 60 et 200 ppp notamment pour une utilisation cosmétique et/ou dermatologique, sur une épaisseur à partir de la face d'application comprise entre 1 et 15 mm.

Avantageusement et selon l'invention, le réservoir présente un volume correspondant à une dose de composition coulante pour un usage unique. Le produit emballé (emballage applicateur + composition coulante) correspondant, utilisé une seule fois et jeté après utilisation, répond à des exigences d'hygiène maximales. Il répond également aux besoins d'une personne temporairement nomade (dans le cadre d'un voyage touristique, professionnel...), et qui souhaite n'emporter avec elle qu'une quantité minimale de ses produits de soin, de beauté, thérapeutiques, etc. correspondant à la quantité normalement consommée durant la période de déplacement (sans avoir à se charger des bouteilles, tubes, ou autres récipients relativement volumineux contenant usuellement lesdits produits).

Avantageusement et selon l'invention, la poire de pompage présente une face intérieure définissant une pluralité de cavités s'ouvrant sur l'ouverture de distribution de façon à former une pluralité de réservoirs séparés fermés par le tampon applicateur. Les réservoirs ainsi formés peuvent être adjacents alignés ou concentriques par exemple. Lorsqu'elle est pincée, ladite poire de pompage refoule simultanément, vers et à travers le tampon, les compositions coulantes contenues dans les différents réservoirs. Un tel emballage applicateur est particulièrement adapté pour délivrer une solution issue du mélange de plusieurs produits (liquides par exemple) réactifs qu'il convient de stocker séparément. Le mélange des différents liquides s'effectue par diffusion des liquides dans le tampon applicateur (par pompage), et se parfait éventuellement lors de l'application des liquides sur la surface à traiter. La mousse d'un tel tampon applicateur est choisie présentant un taux de porosité élevé (définissant un taux de porosité de filtrage) au voisinage immédiat de la face interne afin d'empêcher les liquides de pénétrer dans le tampon et de se mélanger en dehors de toute utilisation du produit emballé. Elle présente avantageusement un taux de porosité faible (définissant un taux de porosité d'application) au voisinage de la face d'application pour faciliter la diffusion et le mélange des liquides réactifs. Un tel emballage applicateur à réservoirs multiples permet également d'emballer une composition hétérogène comprenant par exemple un premier composant sous forme pulvérulente ou préemballée dans des microcapsules, et un deuxième composant porteur liquide (ou crémeux). Une première solution pour emballer selon l'invention une telle composition consiste à réduire la taille des microcapsules ou particules solides du premier composant pour que celle-ci soit inférieure à une taille de pores du tampon qui permette la rétention du composant porteur liquide. Une deuxième solution consiste à traiter le composant liquide en vue d'augmenter sa viscosité de telle sorte qu'un tampon à pores de "grande" taille, apte à laisser passer les microcapsules ou particules solides du premier composant, soit également apte à retenir le composant porteur liquide en l'absence de pompage. Une troisième solution selon l'invention consiste à utiliser un emballage applicateur à réservoirs multiples tel que décrit précédemment, doté de plus d'un tampon applicateur présentant une porosité variable selon des directions orthogonales à la direction transversale (c'est-à-dire variable sur la surface de la face interne du tampon). On réalise, selon l'invention, un tampon applicateur présentant un premier taux de porosité de filtrage compatible avec le composant solide ou encapsulé, en regard d'un premier réservoir, et un second taux de porosité de filtrage compatible avec le composant porteur liquide, en regard d'un second réservoir.

En variante, la poire de pompage présente une face intérieure définissant une pluralité de cavités s'ouvrant sur l'ouverture de distribution de façon à former une pluralité de réservoirs fermés par le tampon applicateur, communiquant entre eux au voisinage de la face interne dudit tampon applicateur. Ce mode de réalisation présente l'avantage de permettre de prévoir un volume de composition coulante inférieur, à surface de tampon à imbiber équivalente. A noter que le mode de réalisation précédent (à réservoirs multiples séparés) présente le même avantage et peut être utilisé pour les mêmes applications que ce dernier mode de réalisation.

Avantageusement et selon l'invention, le tampon applicateur est en une mousse synthétique poreuse à cellules ouvertes, choisie dans le groupe suivant : polyuréthannes; et notamment polyéthers et/ou polyesters, polyéthylènes, chlorures de polyvinyle, mélamines, néoprènes, silicones, polyimides... La nature de la mousse choisie doit être adaptée à l'utilisation de l'emballage, et notamment être chimiquement compatible avec la composition coulante à emballer et avec la surface à traiter. Si la composition coulante est destinée à être appliquée directement sur la peau, la mousse présente également préférentiellement un toucher agréable et doux. Dans une version préférée, le tampon applicateur présente par ailleurs une épaisseur comprise entre 2 et 15 mm, de préférence de l'ordre de 4 mm pour un emballage applicateur enfermant une dose unitaire (pour un usage unique) de composition coulante. Le tampon présente une face d'application de forme quelconque, de préférence circulaire ou elliptique.

Avantageusement et selon l'invention, la poire de pompage est en un matériau synthétique ou naturel souple à température ambiante et adapté pour permettre d'obtenir une poire de pompage étanche à la composition coulante (le matériau est soit étanche à la composition coulante, soit rendu étanche à ladite composition, notamment par plastification, lors de la fabrication de la poire de pompage), choisi dans le groupe suivant : polyéthylènes, polyuréthannes, élastomères tels que caoutchoucs synthétiques ou naturels (latex) trempés... D'autres matériaux thermoplastiques ou thermodurcissables peuvent être employés dans la mesure où ils présentent les propriétés susmentionnées d'étanchéité à la composition coulante et de souplesse à température ambiante. La poire de pompage peut être recouverte d'un habillage (textile par exemple) destiné à en améliorer l'aspect esthétique, le toucher, ou à être utilisé en tant que support d'informations commerciales (support d'une marque, d'un message publicitaire, véhicule d'une image de marque selon l'habillage utilisé...).

Avantageusement et selon l'invention, le tampon applicateur est collé et/ou thermosoudé sur la poire de pompage autour de l'ouverture de distribution.

Dans une version de l'invention, l'emballage applicateur comprend un opercule amovible de protection du tampon applicateur. Un tel opercule ne sert qu'à préserver les qualités hygiéniques du tampon applicateur, et notamment à le protéger de la poussière. Le cas échéant, il prévient également l'évaporation de la composition coulante si celle-ci est volatile, ou son dessèchement notamment dans le cas d'une conservation prolongée du produit emballé (emballage applicateur + composition coulante) avant utilisation. Il n'est en aucun cas utilisé dans le but d'empêcher la composition coulante contenue dans le réservoir de s'écouler spontanément à travers le tampon, cet effet étant obtenu par la combinaison des caractéristiques du tampon applicateur (perméabilité conditionnelle) et de la présence d'une poire de pompage selon l'invention.

L'emballage applicateur selon l'invention est donc caractérisé par la combinaison d'une poire de pompage formant réservoir flexible et d'un tampon applicateur poreux, et est en particulier exempt de moyens mécaniques de rétention de la composition coulante contenue dans le réservoir, autres que le tampon lui-même. Il s'utilise pour emballer la composition coulante telle qu'elle est fabriquée et sera appliquée sur la surface à traiter, sans qu'il ne soit nécessaire de la traiter ou de la transformer préalablement à l'emballage. Un tel emballage est remarquable de simplicité et d'efficacité. L'invention permet ainsi de proposer des emballages applicateurs à coût réduit (faible nombre de pièces et d'opérations nécessaires à leur fabrication et à l'emballage de la composition coulante).

L'invention concerne également un produit emballé comprenant un emballage applicateur tel que défini ci-avant, dont le réservoir, fermé par le tampon applicateur, contient une composition coulante. A noter que, en variante ou en combinaison de l'opercule susmentionné, le produit emballé comprend avantageusement un sachet extérieur, souple ou rigide, de préférence fermé, enveloppant l'emballage applicateur. Un tel sachet peut être transparent ou opaque, uni ou utilisé à titre de support d'inscriptions diverses (marque, slogan, dessin, notice d'utilisation et de mise en garde dans le cas d'une composition coulante thérapeutique...), etc..

L'invention concerne aussi un procédé de fabrication d'un emballage applicateur de composition coulante, conforme à la revendication 23.

Les opérations a/ et b/ sont réalisées indépendamment l'une de l'autre ; elles peuvent être successives chronologiquement, dans un ordre ou dans un autre, ou en variante concomitantes, se dérouler dans un même atelier ou dans deux lieux distincts, etc.

Avantageusement et selon l'invention, pour l'opération a/ :
- on utilise une plaque d'un matériau thermoplastique souple à température ambiante,
- on chauffe ladite plaque thermoplastique jusqu'à une température de son domaine plastique,
- on emboutit la plaque thermoplastique chauffée au moyen d'un poinçon et d'une matrice adaptés pour permettre de former, d'un côté de la plaque, au moins une cavité définissant un réservoir, et, de l'autre côté de la plaque, une protubérance incorporant la(les) cavité(s) et définissant la poire de pompage, (adaptée pour permettre sa préhension et son pincement en vue de permettre de refouler la composition coulante hors du(des) réservoir(s) par l'ouverture de distribution), le matériau thermoplastique et/ou la température de chauffage choisi(e)(s) étant adapté(e)(s) pour que la poire de pompage ainsi formée soit étanche à la composition coulante. Il est à noter que, dans le cas d'une poire de pompage incorporant plusieurs réservoirs, l'ouverture de distribution s'entend comme la réunion -ou l'enveloppe- des ouvertures desdits réservoirs, destinée à être fermée par le tampon applicateur. Selon cette définition, chaque poire de pompage présente une unique ouverture de distribution.

En variante, la poire de pompage est réalisée par moulage (dans un moule ouvert) ou par injection, dans un moule adapté, d'un matériau thermoplastique ou thermodurcissable ou d'un élastomère, puis éventuellement par trempage dudit matériau (notamment dans le cas d'un élastomère).

Pour réaliser le tampon applicateur (opération b/), on utilise préférentiellement une (et une seule) plaque de mousse poreuse, présentant une face frontale destinée à réaliser la face interne du tampon applicateur et une face frontale opposée destinée à réaliser la face d'application du tampon applicateur, en vue de réaliser un tampon applicateur constitué d'une seule pièce en mousse poreuse. Pour ce faire, on peut utiliser une plaque de mousse homogène et/ou uniforme ayant les propriétés de perméabilité décrites précédemment pour la couche de filtrage. En variante, on fabrique une plaque de mousse présentant un gradient de porosité selon une direction, dite direction transversale, au moins sensiblement orthogonale à l'une de ses faces frontales (et qui coïncide avec la direction transversale du tampon applicateur à réaliser telle qu'elle est définie ci-avant). Pour ce faire, avantageusement et selon l'invention :
- on utilise une plaque de mousse synthétique à cellules ouvertes adaptée pour présenter une perméabilité autorisant la diffusion (et notamment la diffusion spontanée) de la composition coulante, ladite plaque de mousse présentant une épaisseur supérieure à celle du tampon applicateur à réaliser,
- on chauffe une face frontale au moins de ladite plaque de mousse au moyen d'une plaque chauffante (la face frontale chauffée est de préférence destinée à être au contact direct et permanent de la composition coulante et à réaliser la face interne du tampon applicateur),
- on comprime la plaque de mousse selon la direction transversale de façon à réduire, sans les colmater, les cellules ouvertes de ladite plaque de mousse sur une fraction de son épaisseur à partir de la face frontale chauffée.

Avantageusement et selon l'invention, on utilise une plaque chauffante portée à une température adaptée pour que la plaque de mousse atteigne, sur une fraction de son épaisseur à partir de la face frontale chauffée, une température de son domaine plastique inférieure à son point de fusion, notamment comprise entre 140 et 240°C dans le cas d'une mousse polyuréthanne.

Avantageusement et selon l'invention, on comprime la plaque de mousse chauffée entre deux plaques de compression au moins sensiblement parallèles espacées d'une distance comprise entre 2 et 15 mm correspondant au moins sensiblement à l'épaisseur du tampon applicateur à réaliser. Dans une version préférée du procédé selon l'invention, l'une au moins des plaques de compression est une plaque chauffante, en vue de mettre en oeuvre concomitamment les étapes de chauffage et de compression de la plaque de mousse.

En variante, pour réaliser un tampon applicateur (opération b/) à gradient de porosité, on superpose et on assemble une pluralité de plaques de mousse ou autres matériaux (papier, textile, plastique microperforé...) ayant des taux de porosité distincts. En variante, on modifie localement (et notamment superficiellement, ou sur une fraction de son épaisseur) les propriétés de perméabilité d'une plaque de mousse choisie perméable à la composition coulante, par greffage d'un polymère sur une face frontale de ladite plaque, ou par colmatage partiel.

L'invention s'étend à un procédé de fabrication d'un produit emballé, caractérisé en ce que l'on réalise une poire de pompage et un tampon applicateur comme précédemment décrit, on dépose une quantité de composition coulante dans chaque cavité (de la poire de pompage), on ferme la(les) cavité(s) au moyen du tampon applicateur, que l'on fixe à la poire de pompage autour de l'ouverture de distribution par thermosoudure (au moyen d'un outil chauffant) ou par collage. Avantageusement et selon l'invention, on injecte la composition coulante dans chaque cavité au moyen d'une seringue.

Avantageusement et selon l'invention, on fabrique simultanément une pluralité d'emballages applicateurs (en un nombre d'opérations identiques à celui nécessaires à la fabrication d'un unique emballage applicateur), selon les étapes suivantes :
- on utilise une plaque de mousse et une plaque de matériau thermoplastique de grandes dimensions (c'est-à-dire de dimensions bien supérieures à celles du tampon applicateur et de la poire de pompage que l'on souhaite réaliser, lesdites plaques devant permettre de réaliser une pluralité de tampons applicateurs et une pluralité de poire de pompage),
- le cas échéant, on chauffe une face frontale au moins de la plaque de mousse et l'on comprime ladite plaque, au moyen d'au moins une plaque chauffante et de plaques de compression présentant des surfaces de chauffage et/ou de compression au moins sensiblement égales ou supérieures à celles des faces frontales de la plaque de mousse, (étant entendu que l'une au moins des plaques de compression peut également être une plaque chauffante en vue de réaliser simultanément les opérations de chauffage et compression),
- on chauffe la plaque thermoplastique jusqu'à une température de son domaine plastique, on emboutit la plaque thermoplastique chauffée au moyen d'un poinçon et d'une matrice adaptés pour permettre de former, d'un côté de ladite plaque, une pluralité de cavités définissant une pluralité de réservoirs, et, de l'autre côté de ladite plaque, une pluralité de protubérances incorporant chacune au moins une cavité (une ou plusieurs, une même poire de pompage pouvant contenir plusieurs réservoirs adjacents) et définissant une pluralité de poires de pompage, adaptées chacune pour permettre sa préhension et son pincement en vue de permettre de refouler la composition coulante hors du(des) réservoir(s) par une ouverture de distribution,
- on dépose une quantité de composition coulante dans chacune des cavités,
- on dispose la plaque de mousse sur la plaque thermoplastique ainsi formée, de façon à fermer les cavités par la plaque de mousse,
- on soude, au moyen d'un outil chauffant, la plaque de mousse et la plaque thermoplastique autour de chaque ouverture de distribution, et l'on découpe les deux plaques autour de chaque soudure ainsi réalisée autour d'une ouverture de distribution. Avantageusement et selon l'invention, on utilise un outil chauffant apte à réaliser simultanément la soudure et le découpage des plaques autour de l'ensemble des ouvertures de distribution (c'est-à-dire apte à réaliser, autour de chacune des poires de pompage, la soudure et le découpage en une seule opération, et également apte à réaliser cette opération simultanément pour toutes les poires de pompage de la plaque).

Avantageusement et selon l'invention, on utilise un bloc de mousse et un bloc de matériau thermoplastique, que l'on débite dans leur épaisseur de façon à obtenir les plaques de mousse et de matériau thermoplastique de grandes dimensions susmentionnées.

Avantageusement et selon l'invention, on fabrique un opercule de protection du tampon applicateur, et on accroche l'opercule de façon amovible sur la poire de pompage et/ou le tampon applicateur, de telle sorte que l'opercule vienne recouvrir la face d'application dudit tampon applicateur.

L'invention concerne également un emballage applicateur, un produit emballé et des procédés de fabrication d'emballages applicateurs et de produits emballés, caractérisés en combinaison par tout ou partie des caractéristiques mentionnées ci-dessus et ci-après.

D'autres buts, caractéristiques et avantages de l'invention apparaîtront à la lecture de la description suivante, qui se réfère aux figures annexées représentant des modes de réalisation préférentiels de l'invention donnés uniquement à titre d'exemples non limitatifs, et dans lesquelles :
- la figure 1 est une vue en perspective d'un premier mode de réalisation d'un produit emballé selon l'invention,
- la figure 2 est une vue en coupe et en perspective du premier mode de réalisation illustré sur la figure 1,
- la figure 3 est une vue en perspective d'un deuxième mode de réalisation d'un produit emballé selon l'invention,
- la figure 4 est une vue en coupe et en perspective du tampon applicateur et de la poire de pompage du premier mode de réalisation, avant assemblage,
- la figure 5 est une vue en perspective de dessous d'une plaque de mousse comprenant une pluralité de tampons applicateurs et d'une plaque préformée comprenant une pluralité de poires de pompage, avant assemblage.

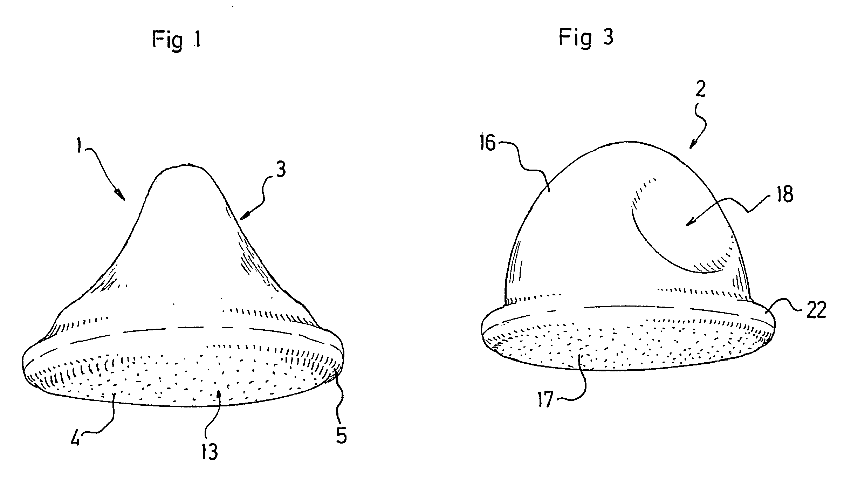

La figure 1 illustre un produit emballé 1, comprenant, selon l'invention, une poire de pompage 3 et un tampon applicateur 4. La poire de pompage est constituée d'une paroi 3a en polyéthylène souple étanche à la composition coulante 8 emballée, présentant une forme de dôme ou de bulbe dont la partie haute, fermée, est légèrement resserrée en cône tronqué, et dont la partie basse évasée est ouverte sur le tampon applicateur 4 (étant précisé que les adjectifs "haute" et "basse" s'entendent en référence aux figures, mais ne définissent pas un sens intrinsèque à l'emballage applicateur). La forme de la paroi 3a est adaptée pour permettre la préhension de l'emballage à l'aide de deux ou trois doigts (dont le pouce) d'une même main, et le pincement manuel de ladite paroi de façon à rapprocher les uns des autres deux ou trois points de ladite paroi situés approximativement dans un même plan horizontal (parallèle au tampon applicateur 4), à mi-hauteur de la paroi 3 a.

Le bord 23 (en partie basse) de cette paroi 3a délimite une ouverture de distribution 19 elliptique, refermée par le tampon applicateur 4. En variante, l'ouverture de distribution peut être circulaire (voir produit emballé 2), polygonale ou autre. La paroi 3a forme un réservoir 7 de réception et de stockage d'une composition coulante 8, liquide ou pâteuse par exemple. Dans cet exemple non limitatif, le réservoir est dimensionné pour recevoir une dose unitaire de composition coulante 8. Typiquement, le réservoir formé présente une hauteur interne de 20 mm (dimension selon l'axe longitudinal 27 de la poire de pompage). Le produit emballé 1 est un produit dit "jetable", à usage unique.

Le tampon applicateur 4 est constitué d'une mousse poreuse à cellules ouvertes en polyuréthanne, présentant une face interne 12 dirigée vers l'intérieur de l'emballage applicateur et du réservoir 7, et une face d'application 13 externe dirigée vers l'extérieur de l'emballage. La face interne 12 est au contact permanent et direct de la composition coulante 8, y compris en dehors de toute utilisation de l'applicateur.

La mousse présente une porosité variable selon sa direction transversale (épaisseur). Le taux de porosité de la mousse, défini comme étant le nombre de pores par unité de longueur selon cette direction, est plus élevé et la taille des pores plus petite au voisinage de la face interne 12 que de la face d'application 13. On distingue ainsi, dans l'épaisseur de la mousse, une première zone 10 de taux de porosité relativement faible, et une deuxième zone 11 de taux de porosité plus élevé agissant comme un filtre conditionnel pour la composition coulante : en l'absence de pincement de la poire de pompage 3, la composition coulante ne pénètre pas dans la zone 11 qui réalise donc une barrière de rétention de ladite composition ; lorsque la poire de pompage est pincée, la composition coulante 8, soumise à une force de compression tendant à la refouler vers l'ouverture de distribution 19 et à l'expulser hors du réservoir 7, pénètre dans la zone 11 et s'écoule jusqu'à la face d'application 13, à la fois sous l'effet du pompage et par diffusion à travers la zone 10. A titre d'exemple, un tel tampon dont le taux de porosité de filtrage (zone 11) est de l'ordre de 300 ppp et le taux de porosité d'application (zone 10) est de l'ordre de 90 ppp, permet d'emballer une composition coulante de viscosité dynamique de l'ordre de 8 Pa.s (mesurée à 25°C). Une pression de pompage supérieure à 100 g/cm² (ou supérieure à 1N/cm²) libère ladite composition coulante.

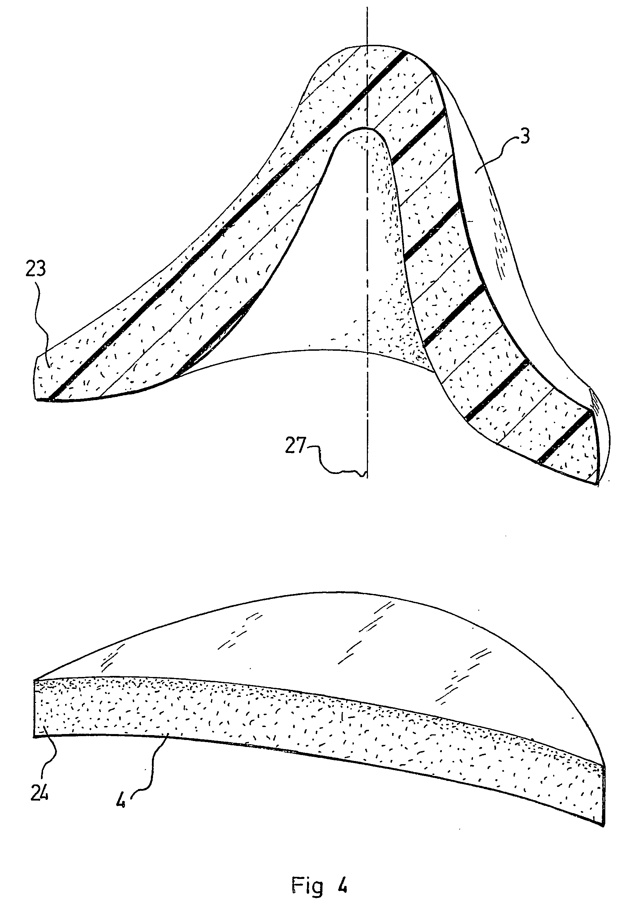

La périphérie 24 du tampon applicateur 4 est fixée sur le bord 23 de la paroi 3a par une soudure périphérique 5. Comme illustré sur la figure 4, avant assemblage, le tampon 4 présente une épaisseur au moins sensiblement constante sur toute sa superficie. Tel est également le cas de la poire de pompage, dont le bord périphérique 23 n'est, après emboutissage, que faiblement compacté. Le tampon applicateur 4 et la poire de pompage 3 sont soudés à leur périphérie 24, 23. Cette soudure est réalisée par chauffage, et compression l'un contre l'autre, du bord 23 de la paroi 3a et de la périphérie 24 du tampon 4. Sous l'effet de la chaleur, la mousse fond au niveau de la périphérie du tampon, les cellules de la mousse se colmatent et se mêlent localement à celles de la paroi 3a. Le bord 23 et la périphérie 24, ainsi compactés et collés, durcissent en se refroidissant. La soudure 5 obtenue est étanche à la composition liquide.

Le tampon applicateur 4 de section elliptique présente un grand axe de l'ordre de 30 mm, un petit d'axe de l'ordre de 26 mm, une épaisseur de 4 mm environ en son centre et de moins d'un millimètre en son bord périphérique au niveau de la soudure 5. La zone 11 de taux de porosité élevé (taux de porosité de filtrage) s'étend sur 0,5 à 1 mm, le taux de porosité devenant très rapidement dégressif au-delà d'un millimètre à partir de la face interne 12. Le tampon applicateur a été réalisé à partir d'une plaque de mousse de 5 mm d'épaisseur, chauffée et comprimée au moyen de plaques de compression d'entrefer de l'ordre de 3,5 mm.

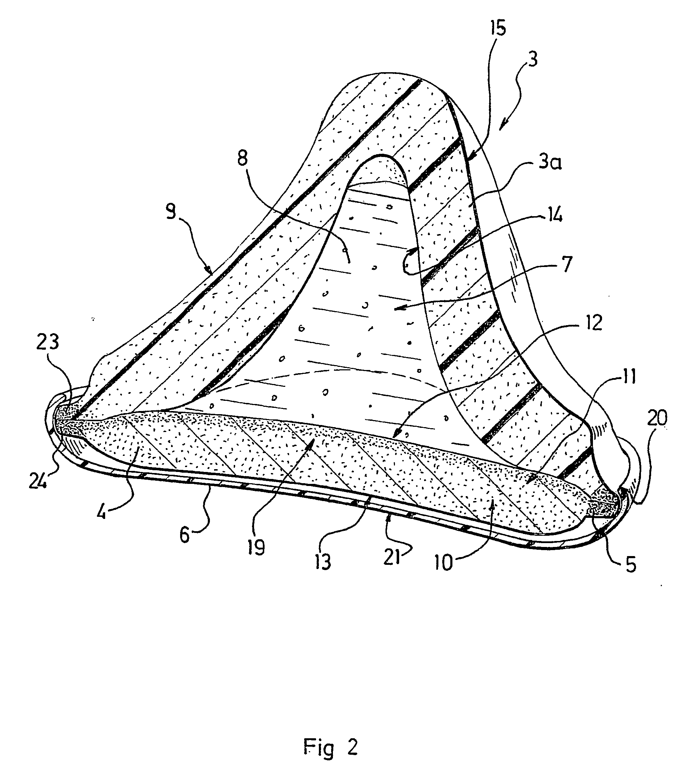

Le produit emballé 1, tel qu'illustré sur la figure 2, est doté d'un opercule 6 de protection du tampon applicateur 4, en matière synthétique rigide. Il présente la forme générale d'une assiette elliptique, avec un fond plat 21 s'étendant, lorsque l'opercule est monté sur la poire de pompage et le tampon applicateur, en regard de la face d'application 13 du tampon, et un bord périphérique 20 recourbé vers le "haut", en direction de la poire de pompage, puis vers le centre de l'opercule de façon à venir envelopper la soudure périphérique 5. L'opercule est aisément placé sur la face d'application 13 du tampon grâce à la flexibilité de la poire de pompage 3 et du tampon applicateur 4.

La figure 3 illustre un autre mode de réalisation d'un produit emballé selon l'invention. Le produit emballé 2 comprend une poire de pompage 16 globalement hémisphérique, et un tampon applicateur 17 en forme de disque, fixés entre eux par une soudure périphérique circulaire 22. La poire de pompage présente deux renfoncements 18 situés sensiblement en regard de part et d'autre d'un plan de symétrie de l'emballage applicateur, à mi-hauteur de la poire de pompage. Les renfoncements 18 sont destinés à accueillir le bout des doigts de l'utilisateur pour faciliter la préhension et le pincement de la poire de pompage 16. Dans un autre mode de réalisation (non illustré), la poire de pompage présente trois renfoncements (pour accueillir le pouce, l'index et le majeur de l'utilisateur).

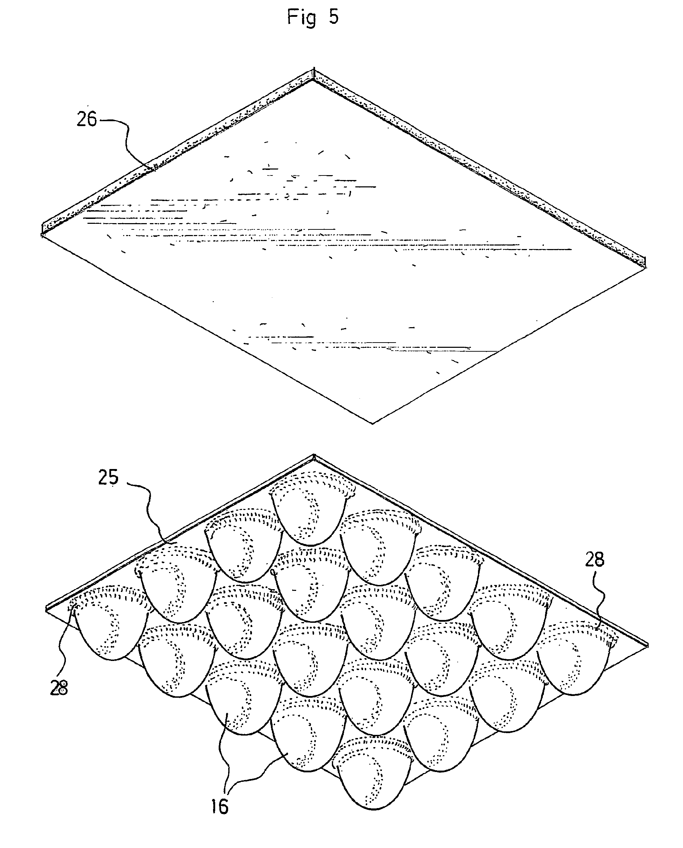

La figure 5 illustre un exemple non limitatif de procédé de fabrication massive et continue d'un tel produit emballé 2. Une série de 20 poires de pompage est formée par emboutissage à chaud d'une plaque 25, initialement plane, d'un matériau thermoplastique, de dimensions initiales sensiblement égales à 160 x 200.

Les poires de pompage sont présentées telles qu'illustrées sur la figure 5, ouvertures de distribution orientées vers le haut, sous une machine d'alimentation en composition coulante adaptée pour injecter une dose de composition coulante dans chaque réservoir. Une plaque de mousse 26, de dimensions équivalentes à celles de la plaque 25, et éventuellement préalablement chauffée et compressée comme précédemment décrit en vue de lui conférer les propriétés de perméabilité/imperméabilité souhaitées selon l'invention, est ensuite disposée sur la plaque 25 de façon à fermer les ouvertures de distribution desdits réservoirs.

Les deux plaques sont assemblées au moyen d'un outil chauffant comprenant une première empreinte inférieure définissant une série de 20 cercles, chaque cercle étant appliqué, autour d'une poire de pompage, sur le bord 28 préformé de ladite poire, et une deuxième empreinte supérieure définissant une série identique de 20 cercles, chaque cercle étant appliqué sur la face supérieure (face d'application) de la plaque 26, en regard d'un cercle de l'empreinte inférieure.

L'outil chauffant est par ailleurs adapté pour découper les deux plaques autour de chaque soudure ainsi réalisée.

Il va de soi que l'invention peut faire l'objet de nombreuses variantes par rapport aux modes de réalisation précédemment décrits et représentés sur les figures. En particulier, toute autre forme de la poire de pompage, dont l'ergonomie conduit à une meilleure préhension et à un pompage facilité, et tout autre matériau utilisé pour ladite poire, est conforme à l'invention. De même, tout autre procédé de fabrication de la poire de pompage (moulage ...) est conforme à l'invention.

Par ailleurs, il est possible de réaliser un tampon applicateur à gradient de porosité par superposition et assemblage de plaques ou feuilles de matériaux (mousses ou autres) de porosités différentes.

## Revendications

1. Emballage applicateur (1) de composition coulante, comprenant :
- une poire de pompage (3) manuel formant un réservoir étanche (7) de stockage de la composition coulante (8) et présentant une ouverture (19) de distribution de ladite composition coulante, ladite poire de pompage étant adaptée pour permettre sa préhension et son pincement manuel en vue de refouler la composition coulante hors du réservoir par l'ouverture de distribution,
- un dispositif applicateur (4) présentant une face interne (12) adaptée pour combler l'ouverture de distribution de façon à venir au contact direct de la composition coulante contenue dans le réservoir, et une face externe (13) opposée; dite face d'application, adaptée pour permettre l'application de la composition coulante (8) sur une surface à traiter, ledit dispositif applicateur (4) étant adapté pour autoriser la diffusion de la composition coulante jusqu'à la face d'application (13) lorsque la poire de pompage (3) est pincée,
**caractérisé en ce que** le dispositif applicateur est constitué d'un tampon applicateur en une seule pièce comprenant au moins une couche (11), dite couche de filtrage, continue, en matière perméable à la composition coulante lorsque la poire de pompage (3) est pincée, et imperméable à la composition coulante en l'absence de pincement de la poire de pompage (3).

2. Emballage applicateur selon la revendication 1, **caractérisé en ce que** la couche de filtrage (11) s'étend sur une fraction au moins de l'épaisseur du tampon applicateur (4) à partir de la face interne (12).

3. Emballage applicateur selon l'une des revendications 1 à 2, **caractérisé en ce que** le tampon applicateur (4) est en mousse poreuse.

4. Emballage applicateur selon la revendication 3, **caractérisé en ce que** la mousse du tampon applicateur (4) présente, sur une fraction au moins de son épaisseur et en regard d'une portion au moins de l'ouverture de distribution, une porosité comprise entre 10 et 1000 ppp.

5. Emballage applicateur selon l'une des revendications 3 ou 4, **caractérisé en ce que** la mousse du tampon applicateur (4) présente une porosité variable selon une direction, dite direction transversale, au moins sensiblement orthogonale à la face d'application (13) et/ou à la face interne (12).

6. Emballage applicateur selon la revendication 5, **caractérisé en ce que** la mousse du tampon applicateur présente, sur une fraction (11) de son épaisseur à partir de la face interne (12), un taux de porosité élevé, dit taux de porosité de filtrage, adapté pour empêcher la pénétration de la composition coulante (8) dans le tampon applicateur en l'absence de pincement de la poire de pompage (3), et, sur une fraction (10) de son épaisseur à partir de la face d'application (13), un taux de porosité plus faible, dit taux de porosité d'application, compatible avec la surface à traiter.

7. Emballage applicateur selon la revendication 6, pour composition coulante de viscosité dynamique comprise entre 0,5.10⁻³ et 5 Pa.s, **caractérisé en ce que** la mousse du tampon applicateur présente un taux de porosité de filtrage compris entre 500 et 1000 ppp sur une épaisseur à partir de la face interne comprise entre 0,1 et 15 mm.

8. Emballage applicateur selon la revendication 6, pour composition coulante de viscosité dynamique comprise entre 5 et 15 Pa.s, **caractérisé en ce que** la mousse du tampon applicateur présente un taux de porosité de filtrage compris entre 60 et 500 ppp sur une épaisseur à partir de la face interne comprise entre 0,1 et 15 mm.

9. Emballage applicateur selon la revendication 6, pour composition coulante de viscosité dynamique comprise entre 15 et 1000 Pa.s, **caractérisé en ce que** la mousse du tampon applicateur présente un taux de porosité de filtrage compris entre 10 et 60 ppp sur une épaisseur à partir de la face interne comprise entre 0,1 et 15 mm.

10. Emballage applicateur selon l'une des revendications 6 à 9, **caractérisé en ce que** la mousse du tampon applicateur présente un taux de porosité d'application compris entre 10 et 200 ppp sur une épaisseur à partir de la face d'application comprise entre 1 et 15 mm.

11. Emballage applicateur selon l'une des revendications 1 à 10, **caractérisé en ce que** le réservoir (7) présente un volume correspondant à une dose de composition coulante (8) pour un usage unique.

12. Emballage applicateur selon l'une des revendications 1 à 11, **caractérisé en ce que** la poire de pompage présente une face intérieure définissant une pluralité de cavités s'ouvrant sur l'ouverture de distribution de façon à former une pluralité de réservoirs séparés fermés par le tampon applicateur.

13. Emballage applicateur selon l'une des revendications 1 à 11, **caractérisé en ce que** la poire de pompage présente une face intérieure définissant une pluralité de cavités s'ouvrant sur l'ouverture de distribution de façon à former une pluralité de réservoirs fermés par le tampon applicateur, communiquant entre eux au voisinage de la face interne dudit tampon applicateur.

14. Emballage applicateur selon l'une des revendications 1 à 13, **caractérisé en ce que** le tampon applicateur (4) est en une mousse synthétique poreuse à cellules ouvertes, choisie dans le groupe suivant : polyuréthannes, et notamment polyéthers et/ou polyesters, polyéthylènes, chlorures de polyvinyle, mélamines, néoprènes, silicones, polyimides.

15. Emballage applicateur selon l'une des revendications 1 à 14, **caractérisé en ce que** le tampon applicateur (4) présente une épaisseur comprise entre 2 et 15 mm.

16. Emballage applicateur selon les revendications 11 et 15, **caractérisé en ce que** le tampon applicateur (4) présente une épaisseur de l'ordre de 4 mm.

17. Emballage applicateur selon l'une des revendications 1 à 16, **caractérisé en ce que** la poire de pompage (3) est en un matériau synthétique ou naturel souple à température ambiante et adapté pour permettre d'obtenir une poire de pompage étanche à la composition coulante, choisi dans le groupe suivant : polyéthylènes, polyuréthannes, élastomères tels que caoutchoucs synthétiques ou naturels trempés.

18. Emballage applicateur selon l'une des revendications 1 à 17, **caractérisé en ce que** la poire de pompage est recouverte d'un habillage.

19. Emballage applicateur selon l'une des revendications 1 à 18, **caractérisé en ce que** le tampon applicateur (4) est collé et/ou thermosoudé sur la poire de pompage (3) autour de l'ouverture de distribution (19).

20. Emballage applicateur selon l'une des revendications 1 à 19, **caractérisé en ce qu'**il comprend un opercule (6) amovible de protection du tampon applicateur (4).

21. Produit emballé **caractérisé en ce qu'**il comprend un emballage applicateur (1) selon l'une des revendications 1 à 20 dont le réservoir (7), fermé par le tampon applicateur (4), contient une composition coulante (8).

22. Produit emballé selon la revendication 21, **caractérisé en ce qu'**il comprend un sachet extérieur enveloppant l'emballage applicateur.

23. Procédé de fabrication d'un emballage applicateur de composition coulante, comprenant les deux opérations suivantes :
a/ - on réalise une poire de pompage manuel (3) de telle sorte que ladite poire forme un réservoir étanche de stockage de la composition coulante, présente une ouverture de distribution de ladite composition coulante, et soit adaptée pour permettre sa préhension et son pincement manuel en vue de permettre de refouler la composition coulante hors du réservoir par l'ouverture de distribution,
b/ - on réalise un dispositif applicateur (4) présentant une face interne (12) apte à venir combler l'ouverture de distribution de la poire de pompage de façon à venir au contact direct de la composition coulante, et une face externe opposée (13), dite face d'application, adaptée pour permettre l'application de la composition coulante sur une surface à traiter, ledit dispositif applicateur (4) étant réalisé pour pouvoir autoriser la diffusion de la composition coulante jusqu'à la face d'application (13) lorsque la poire de pompage (3) est pincée,
**caractérisé en ce que** dans l'opération b/ on réalise le dispositif applicateur dudit emballage applicateur constitué d'un tampon applicateur d'une seule pièce et comprenant au moins une couche (11), dite couche de filtrage, continue en une matière adaptée pour pouvoir être perméable à la composition coulante (8) lorsque la poire de pompage (3) est pincée et imperméable à la composition coulante en l'absence de pincement de la poire de pompage.

24. Procédé selon la revendication 23, **caractérisé en ce que**, dans l'opération a/ :
- on utilise une plaque d'un matériau thermoplastique souple à température ambiante,
- on chauffe ladite plaque thermoplastique jusqu'à une température de son domaine plastique,
- on emboutit la plaque thermoplastique chauffée au moyen d'un poinçon et d'une matrice adaptés pour permettre de former, d'un côté de la plaque, au moins une cavité (7) définissant chacune un réservoir, et, de l'autre côté de la plaque, une protubérance incorporant la (les) cavité(s) et définissant la poire de pompage (3), le matériau thermoplastique et/ou la température de chauffage choisi(e)(s) étant adapté(e)(s) pour que la poire de pompage ainsi formée soit étanche à la composition coulante.

25. Procédé selon l'une des revendications 23 ou 24, **caractérisé en ce que**, dans l'opération b/, on utilise une plaque de mousse poreuse présentant une face frontale destinée à réaliser la face interne (12) du tampon applicateur et une face frontale opposée destinée à réaliser la face d'application (13) du tampon applicateur, en vue de réaliser un tampon applicateur (4) constitué d'une seule pièce en mousse poreuse.

26. Procédé selon la revendication 25, **caractérisé en ce que** :
- on utilise une plaque de mousse synthétique à cellules ouvertes, adaptée pour présenter une perméabilité autorisant la diffusion de la composition coulante, et présentant une épaisseur supérieure à celle du tampon applicateur à réaliser,
- on chauffe une face frontale au moins de ladite plaque de mousse au moyen d'une plaque chauffante,
- on comprime la plaque de mousse selon une direction, dite direction transversale, au moins sensiblement orthogonale à l'une de ses faces frontales, de façon à réduire, sans les colmater, les cellules ouvertes de ladite plaque de mousse sur une fraction de son épaisseur à partir de la face frontale chauffée.

27. Procédé selon la revendication 26, **caractérisé en ce que** l'on utilise une plaque chauffante portée à une température adaptée pour que la plaque de mousse atteigne, sur une fraction de son épaisseur à partir de la face frontale chauffée, une température de son domaine plastique inférieure à son point de fusion, notamment comprise entre 140 et 240°C.

28. Procédé selon l'une des revendications 26 ou 27, **caractérisé en ce que** l'on comprime la plaque de mousse chauffée entre deux plaques de compression au moins sensiblement parallèles espacées d'une distance comprise entre 2 et 15 mm correspondant au moins sensiblement à l'épaisseur du tampon applicateur (4) à réaliser.

29. Procédé selon la revendication 28, **caractérisé en ce que** l'une au moins des plaques de compression est une plaque chauffante, en vue de mettre en oeuvre concomitamment les étapes de chauffage et de compression de la plaque de mousse.

30. Procédé de fabrication d'un produit emballé, **caractérisé en ce que** l'on réalise une poire de pompage (3) et un tampon applicateur (4) selon l'une des revendications 23 à 29, on dépose une quantité de composition coulante (8) dans chaque cavité, on ferme la(les) cavité(s) au moyen du tampon applicateur, que l'on fixe à la poire de pompage autour de l'ouverture de distribution par thermosoudure ou par collage.

31. Procédé selon la revendication 30, **caractérisé en ce que** l'on injecte la composition coulante dans chaque cavité au moyen d'une seringue.

32. Procédé selon l'une des revendications 30 ou 31, **caractérisé en ce que**:
- on utilise une plaque de mousse et une plaque de matériau thermoplastique de grandes dimensions,
- le cas échéant, on chauffe une face frontale au moins de la plaque de mousse et l'on comprime ladite plaque, au moyen d'au moins une plaque chauffante et de plaques de compression présentant des surfaces de chauffage et/ou de compression au moins sensiblement égales ou supérieures à celles des faces frontales de la plaque de mousse,
- on chauffe la plaque thermoplastique jusqu'à une température de son domaine plastique, on emboutit la plaque thermoplastique chauffée au moyen d'un poinçon et d'une matrice adaptés pour permettre de former, d'un côté de ladite plaque, une pluralité de cavités définissant une pluralité de réservoirs, et, de l'autre côté de ladite plaque, une pluralité de protubérances incorporant chacune au moins une cavité et définissant une pluralité de poires de pompage, adaptées chacune pour permettre sa préhension et son pincement en vue de permettre de refouler la composition coulante hors du(des) réservoir(s) par une ouverture de distribution,
- on dépose une quantité de composition coulante dans chacune des cavités,
- on dispose la plaque de mousse sur la plaque thermoplastique ainsi formée, de façon à fermer les cavités par la plaque de mousse,
- on soude, au moyen d'un outil chauffant, la plaque de mousse et la plaque thermoplastique autour de chaque ouverture de distribution, et l'on découpe les deux plaques autour de chaque soudure ainsi réalisée autour d'une ouverture de distribution.

33. Procédé selon la revendication 32, **caractérisé en ce que** l'on utilise un outil chauffant apte à réaliser simultanément la soudure et le découpage des plaques autour de l'ensemble des ouvertures de distribution.

34. Procédé selon l'une des revendications 30 à 33, **caractérisé en ce que** l'on fabrique un opercule de protection du tampon applicateur, et l'on accroche l'opercule de façon amovible sur la poire de pompage et/ou le tampon applicateur, de telle sorte que l'opercule vienne recouvrir la face d'application dudit tampon applicateur.

## Claims

1. Applicator packaging (1) for a fluid composition, comprising:
- a manual pumping bulb (3) which forms a sealed storage reservoir (7) for the fluid composition (8) and has an opening (19) for distribution of the said fluid composition, the said pumping bulb being designed such as to make it possible to grasp and pinch it manually in order to force the fluid composition out of the reservoir via the distribution opening;
- an applicator device (4) with an inner surface (12) which is designed to fill in the distribution opening so as to come into direct contact with the fluid composition contained in the reservoir, and an opposite outer surface (13), known as the application surface, which is designed to permit application of the fluid composition (8) onto a surface to be treated, the said applicator device (4) being designed to permit diffusion of the fluid composition as far as the application surface (13) when the pumping bulb (3) is pinched,
**characterised in that** the applicator device consists of an applicator pad in a single piece comprising at least one continuous layer (11), known as the filtering layer, made of material which is permeable to the fluid composition when the pumping bulb (3) is pinched, and is impermeable to the fluid composition in the absence of pinching of the pumping bulb (3).

2. Applicator packaging according to claim 1,
**characterised in that** the filtering layer (11) extends over at least a fraction of the thickness of the applicator pad (4), starting from the inner surface (12).

3. Applicator packaging according to claim 1 or claim 2,
**characterised in that** the applicator pad (4) is made of porous foam (4).

4. Applicator packaging according to claim 3,
**characterised in that** the foam of the applicator pad (4) has porosity of between 10 and 1000 ppp, along at least a fraction of its thickness and opposite at least a portion of the distribution opening.

5. Applicator packaging according to claim 3 or claim 4,
**characterised in that** the foam of the applicator pad (4) has porosity which is variable in one direction, known as the transverse direction, which is at least substantially at right-angles to the application surface (13) and/or to the inner surface (12).

6. Applicator packaging according to claim 5,
**characterised in that** the foam of the applicator pad has, along a fraction (11) of its thickness starting from the inner surface (12), a high level of porosity, known as the filtering porosity level, which is designed to prevent penetration of the fluid composition (8) into the applicator pad in the absence of pinching of the pumping bulb (3), and, along a fraction (10) of its thickness starting from the application surface (13), it has a lower level of porosity, known as the application porosity level, which is compatible with the surface to be treated.

7. Applicator packaging according to claim 6, for a fluid composition with dynamic viscosity of between 0.5.10⁻³ and 5 Pa.s, **characterised in that** the foam of the applicator pad has a level of filtering porosity of between 500 and 1000 ppp over a thickness of between 0.1 and 15 mm, starting from the inner surface.

8. Applicator packaging according to claim 6, for a fluid composition with dynamic viscosity of between 5 and 15 Pa.s, **characterised in that** the foam of the applicator pad has a level of filtering porosity of between 60 and 500 ppp over a thickness of between 0.1 and 15 mm, starting from the inner surface.

9. Applicator packaging according to claim 6, for a fluid composition with dynamic viscosity of between 15 and 1000 Pa.s, **characterised in that** the foam of the applicator pad has a level of filtering porosity of between 10 and 60 ppp over a thickness of between 0.1 and 15 mm, starting from the inner surface.

10. Applicator packaging according to one of claims 6 to 9, **characterised in that** the foam of the applicator pad has a level of filtering porosity of between 10 and 200 ppp over a thickness of between 1 and 15 mm, starting from the application surface.

11. Applicator packaging according to one of claims 1 to 10, **characterised in that** the reservoir (7) has a volume corresponding to a dose of fluid composition (8) for single use.

12. Applicator packaging according to one of claims 1 to 11, **characterised in that** the pumping bulb has an inner surface which defines a plurality of cavities which open onto the distribution opening, such as to form a plurality of separate reservoirs which are closed by the applicator pad.

13. Applicator packaging according to one of claims 1 to 11, **characterised in that** the pumping bulb has an inner surface which defines a plurality of cavities which open onto the distribution opening, such as to form a plurality of reservoirs which are closed by the applicator pad, and communicate with one another in the vicinity of the inner surface of the said applicator pad.

14. Applicator packaging according to one of claims 1 to 13, **characterised in that** the applicator pad (4) is made of porous synthetic foam with open cells, selected from the following group: polyurethanes, and in particular polyethers and/or polyesters, polyethylenes, polyvinyl chlorides, melamines, neoprenes, silicones and polyimides.

15. Applicator packaging according to one of claims 1 to 14, **characterised in that** the applicator pad (4) has a thickness of between 2 and 15 mm.

16. Applicator packaging according to claims 11 and 15, **characterised in that** the applicator pad (4) has a thickness of approximately 4 mm.

17. Applicator packaging according to one of claims 1 to 16, **characterised in that** the pumping bulb (3) is made of a natural or synthetic material which is flexible at ambient temperature, is designed to make it possible to obtain a pumping bulb which is sealed against the fluid composition, and is selected from the following group: polyethylenes, polyurethanes, and elastomers such as hardened natural or synthetic rubbers.

18. Applicator packaging according to one of claims 1 to 17, **characterised in that** the pumping bulb is covered with a covering.

19. Applicator packaging according to one of claims 1 to 18, **characterised in that** the applicator pad (4) is glued and/or heat sealed onto the pumping bulb (3) around the distribution opening (19).

20. Applicator packaging according to one of claims 1 to 19, **characterised in that** it comprises a removable cover (6) for protection of the applicator pad (4).

21. Packaged product, **characterised in that** it comprises applicator packaging (1) according to one of claims 1 to 20, the reservoir (7) of which, which is closed by the applicator pad (4), contains a fluid composition (8).

22. Packaged product according to claim 21, **characterised in that** it comprises an outer bag which envelopes the applicator packaging.

23. Method for production of an applicator packaging for a fluid composition, comprising the following two operations:
a. A manual pumping bulb (3) is provided, such that the said bulb forms a sealed storage reservoir for the fluid composition, has an opening for distribution of the said fluid composition, and is designed such as to make it possible to grasp and pinch the bulb manually in order to allow the fluid composition to be forced out of the reservoir via the distribution opening;
b. An applicator device (4) is provided, with an inner surface (12) which can fill in the distribution opening of the pumping bulb, so as to come into direct contact with the fluid composition, and with an opposite outer surface (13), known as the application surface, which is designed to permit application of the fluid composition onto a surface to be treated, the said applicator device (4) being designed to permit diffusion of the fluid composition as far as the application surface (13) when the pumping bulb (3) is pinched,
**characterised in that**, in the operation b., the applicator device of the said applicator packaging consists of an applicator pad in a single piece comprising at least one continuous layer (11), known as the filtering layer, made of material which is designed to be able to be permeable to the fluid composition (8) when the pumping bulb (3) is pinched, and impermeable to the fluid composition in the absence of pinching of the pumping bulb.

24. Method according to claim 23, **characterised in that**, in the operation a.:
- a sheet of thermoplastic material which is flexible at ambient temperature is used;
- the said thermoplastic sheet is heated to a temperature of its plastic domain;
- the heated thermoplastic sheet is stamped by means of a punch and a die which are designed to make it possible to form, on one side of the sheet, at least one cavity (7), each of which defines a reservoir, and, on the other side of the sheet, to form a protuberance which incorporates the cavity/cavities, and defines the pumping bulb (3), the thermoplastic material and/or the heating temperature selected being designed such that the pumping bulb thus formed is sealed against the fluid composition.

25. Method according to one of claims 23 or 24,
**characterised in that**, in the operation b., a sheet of porous foam is used with a front surface which is designed to provide the inner surface (12) of the applicator pad, and an opposite front surface which is designed to provide the application surface (13) of the applicator pad, in order to provide an applicator pad (4) constituted by a single piece of porous foam.

26. Method according to claim 25, **characterised in that**:
a sheet of synthetic foam with open cells is used, which is designed to have permeability which permits diffusion of the fluid composition, and has a thickness greater than that of the applicator pad to be produced;
- at least a front surface of the said sheet of foam is heated by means of a heating plate;
- the sheet of foam is compressed in a direction, known as the transverse direction, which is at least substantially at right-angles to one of its front surfaces, such as to reduce the open cells of the said sheet of foam, without blocking them, over a fraction of the thickness of the foam, starting from the heated front surface.

27. Method according to claim 26, **characterised in that** a heating plate is used which is brought up to a temperature suitable for the sheet of foam to reach, over a fraction of its thickness starting from the heated front surface, a temperature of its plastic domain which is lower than its melting point, and in particular is between 140 and 240°C.

28. Method according to claim 26 or 27, **characterised in that** the heated sheet of foam is compressed between two at least substantially parallel compression plates which are spaced by a distance of between 2 and 15 mm, corresponding at least substantially to the thickness of the applicator pad (4) to be produced.

29. Method according to claim 28, **characterised in that** at least one of the compression plates is a heating plate, in order to implement simultaneously the steps of heating and compression of the sheet of foam.

30. Method for production of a packaged product,
**characterised in that** a pumping bulb (3) and an applicator pad (4) according to one of claims 23 to 29 are produced, a quantity of fluid composition (8) is deposited in each cavity, and the said cavities are closed by means of an applicator pad which is secured to the pumping bulb around the distribution opening by being heat sealed or glued.

31. Method according to claim 30, **characterised in that** the fluid composition is injected into each cavity by means of a syringe.

32. Method according to claim 30 or 31, **characterised in that**:
- a sheet of foam and a sheet of thermoplastic material with large dimensions are used;
- if applicable, at least a front surface of the sheet of foam is heated, and the said sheet is compressed by means of at least one heating plate and compression plates which have heating and/or compression surfaces which are at least substantially the same size as, or larger than, those of the front surface of the sheet of foam;
- the thermoplastic sheet is heated to a temperature of its plastic domain, the heated thermoplastic sheet is stamped by means of a punch and a die which are suitable for making it possible to form on one side of the said sheet a plurality of cavities which define a plurality of reservoirs, and to form on the other side of the said sheet a plurality of protuberances which each incorporate at least one cavity, and define a plurality of pumping bulbs, each of which is designed to be able to be grasped and pinched in order to make it possible to force the fluid composition out of the reservoir(s) via a distribution opening;
- a quantity of fluid composition is deposited in each of the cavities;
- the sheet of foam is disposed on the thermoplastic sheet thus formed, such as to close the cavities by means of the sheet of foam; and
- by means of a heating tool the sheet of foam and the thermoplastic sheet are welded around each distribution opening, and the two sheets are cut around each weld thus produced, around a distribution opening.

33. Method according to claim 32, **characterised in that** a heating tool is used which can carry out simultaneously the welding and cutting of the sheets around the assembly of the distribution openings.

34. Method according to one of claims 30 to 33,
**characterised in that** a protective cap for the applicator pad is produced, and the cap is connected in a removable manner to the pumping bulb and/or the applicator pad, such that the cover covers the application surface of the said applicator pad.

## Patentansprüche

1. Verpackung zum Aufbringen (1) einer fließfähigen Zusammensetzung, die folgende Teile umfaßt:
- Einen manuell betätigten Pumpenbalg (3), der einen dichten Lagerbehälter (7) für die fließfähige Zusammensetzung (8) bildet und eine Abgabeöffnung (19) der besagten fließfähigen Zusammensetzung aufweist, wobei der besagte Pumpenbalg so angepaßt ist, daß er ergriffen und manuell zusammengekniffen werden kann, damit die fließfähige Zusammensetzung durch die Abgabeöffnung aus dem Behälter befördert werden kann;
- Einen Applikator (4), der eine Innenseite (12) aufweist, die so angepaßt ist, daß sie die Abgabeöffnung verschließt und somit in direkte Berührung mit der im Behälter enthaltenen fließfähigen Zusammensetzung gelangt, und eine entgegengesetzte Außenseite (13), die sogenannte Applikationsseite, die so angepaßt ist, daß das Aufbringen der fließfähigen Zusammensetzung (8) auf einer zu behandelnden Fläche möglich wird, wobei der besagte Applikator (4) angepaßt ist, um die Verbreitung der fließfähigen Zusammensetzung bis zur Applikationsseite (13) zuzulassen, wenn der Pumpenbalg (3) zusammengekniffen wird,
**dadurch gekennzeichnet, daß** der Applikator aus einem Applikatorkissen in einem einzigen Teil besteht, das mindestens eine Schicht (11) umfaßt, die sogenannte kontinuierliche Filterschicht aus für die fließfähige Zusammensetzung durchlässigem Material, wenn der Pumpenbalg (3) zusammengekniffen wird, und für die fließfähige Zusammensetzung undurchlässig, wenn der Pumpenbalg (3) nicht zusammengekniffen wird.

2. Verpackung für Applikation nach Anspruch 1, **dadurch gekennzeichnet, daß** sich die Filterschicht (11) auf einem Teil mindestens mit der Dicke des Applikatorkissens (4) ab der Innenseite (12) erstreckt.

3. Verpackung für Applikation nach einem der Ansprüche 1 bis 2, **dadurch gekennzeichnet, daß** das Applikatorkissen (4) aus porösem Schaumstoff besteht.

4. Verpackung für Applikation nach Anspruch 3, **dadurch gekennzeichnet, daß** der Schaumstoff des Applikatorkissens (4) mindestens auf einem Teil seiner Dicke und vor einem Abschnitt der Abgabeöffnung eine Porosität von zwischen 10 und 1.000 ppp aufweist.

5. Verpackung für Applikation nach einem der Ansprüche 3 oder 4, **dadurch gekennzeichnet, daß** der Schaumstoff des Applikatorkissens (4) eine variable Porosität in einer Richtung, der sogenannten Querrichtung, aufweist, mindestens deutlich orthogonal zur Applikationsseite (13) und/oder zur Innenseite (12).

6. Verpackung für Applikation nach Anspruch 5, **dadurch gekennzeichnet, daß** der Schaumstoff des Applikatorkissens (4) auf einem Teil (11) seiner Dicke ab der Innenseite (12) einen hohen Porositätssatz aufweist, den sogenannten Filterporositätssatz, der so angepaßt ist, daß das Eindringen der fließfähigen Zusammensetzung (8) in das Applikatorkissen bei fehlendem Zusammenkneifen des Pumpenbalgs (3) verhindert wird, und auf einem Teil (10) seiner Dicke ab der Applikationsseite (13 einen geringeren Porositätssatz, den sogenannten Applikations-Porositätssatz, der mit der zu behandelnden Oberfläche verträglich ist.

7. Verpackung für Applikation nach Anspruch 6 für fließfähige Zusammensetzung mit dynamischer Viskosität von zwischen 0,5.10⁻³ und 5 Pa.s, **dadurch gekennzeichnet, daß** der Schaumstoff des Applikatorkissens einen Filterporositätssatz von zwischen 500 und 1.000 ppp auf einer Dicke ab der Innenseite von zwischen 0,1 und 15 Millimeter aufweist.

8. Verpackung für Applikation nach Anspruch 6 für fließfähige Zusammensetzung mit dynamischer Viskosität von zwischen 5 und 15 Pa.s, **dadurch gekennzeichnet, daß** der Schaumstoff des Applikatorkissens einen Filterporositätssatz zwischen 60 und 500 ppp auf einer Dicke ab der Innenseite von zwischen 0,1 und 15 Millimeter aufweist.

9. Verpackung für Applikation nach Anspruch 6 für fließfähige Zusammensetzung mit dynamischer Viskosität von zwischen 15 und 1.000 Pa.s, **dadurch gekennzeichnet, daß** der Schaumstoff des Applikatorkissens einen Filterporositätssatz von zwischen 10 und 60 ppp auf einer Dicke ab der Innenseite von zwischen 0,1 und 15 Millimeter aufweist.

10. Verpackung für Applikation nach einem beliebigen der vorstehenden Ansprüche 6 bis 9, **dadurch gekennzeichnet, daß** der Schaumstoff des Applikatorkissens einen Applikations-Porositätssatz von zwischen 10 und 200 ppp auf einer Dicke ab der Applikationsseite von zwischen 1 und 15 Millimeter aufweist.

11. Verpackung für Applikation nach einem beliebigen der vorstehenden Ansprüche 1 bis 10, **dadurch gekennzeichnet, daß** der Behälter (7) ein Volumen aufweist, das einer Dosis fließfähige Zusammensetzung (8) für einen Einmalgebrauch entspricht.

12. Verpackung für Applikation nach einem beliebigen der vorstehenden Ansprüche 1 bis 11, **dadurch gekennzeichnet, daß** der Pumpenbalg eine Innenseite aufweist, die eine Vielzahl Austiefungen definiert, die sich beim Öffnen der Abgabeöffnung öffnen, so daß sie eine Vielzahl von getrennten Behältern bilden, die vom Applikatorkissen geschlossen werden.

13. Verpackung für Applikation nach einem beliebigen der vorstehenden Ansprüche 1 bis 11, **dadurch gekennzeichnet, daß** der Pumpenbalg eine Innenseite aufweist, die eine Vielzahl Austiefungen definiert, die sich beim Öffnen der Abgabeöffnung öffnen, so daß sie eine Vielzahl von Behältern bilden, die vom Applikatorkissen geschlossen werden und in der Nähe der Innenseite des besagten Applikatorkissens miteinander in Verbindung stehen.

14. Verpackung für Applikation nach einem beliebigen der vorstehenden Ansprüche 1 bis 13, **dadurch gekennzeichnet, daß** das Applikatorkissen (4) aus einem porösen synthetischen Schaumstoff mit offenen Zellen besteht, der aus folgender Gruppe gewählt wird: Polyurethane und insbesondere Polyether und/oder Polyester, Polyethylene, Polyvinylchloride, Melamine, Neoprene, Silikone, Polyimide.

15. Verpackung für Applikation nach einem beliebigen der vorstehenden Ansprüche 1 bis 14, **dadurch gekennzeichnet, daß** das Applikatorkissen (4) eine Dicke von zwischen 2 und 15 Millimeter aufweist.

16. Verpackung für Applikation nach den vorstehenden Ansprüchen 11 und 15, **dadurch gekennzeichnet, daß** das Applikatorkissen (4) eine Dicke von 4 Millimeter aufweist.

17. Verpackung für Applikation nach einem beliebigen der vorstehenden Ansprüche 1 bis 16, **dadurch gekennzeichnet, daß** der Pumpenbalg (3) aus einem synthetischen oder natürlichen bei Raumtemperatur weichen Material besteht, mit dem ein gegen die fließfähige Zusammensetzung dichter Pumpenbalg erzielt werden kann, und das aus folgender Gruppe gewählt wird: Polyethylene, Polyurethane, Elastomere, wie zum Beispiel gehärtete synthetische oder natürliche Gummis.

18. Verpackung für Applikation nach einem beliebigen der vorstehenden Ansprüche 1 bis 17, **dadurch gekennzeichnet, daß** der Pumpenbalg mit einer Verkleidung überzogen ist.

19. Verpackung für Applikation nach einem beliebigen der vorstehenden Ansprüche 1 bis 18, **dadurch gekennzeichnet, daß** das Applikatorkissen (4) auf den Pumpenbalg (3) um die Abgabeöffnung (19) herum geklebt und/oder wärmegeschweißt ist.

20. Verpackung für Applikation nach einem beliebigen der vorstehenden Ansprüche 1 bis 19, **dadurch gekennzeichnet, daß** sie einen abnehmbaren Deckel (6) zum Schutz des Applikatorkissens (4) umfaßt.

21. Verpacktes Produkt, **dadurch gekennzeichnet, daß** es eine Verpackung für Applikation (1) nach einem beliebigen der vorstehenden Ansprüche 1 bis 20 umfaßt, dessen vom Applikatorkissen (4) geschlossener Behälter (7) eine fließfähige Zusammensetzung enthält.

22. Verpacktes Produkt nach Anspruch 21, **dadurch gekennzeichnet, daß** es eine äußere Tüte umfaßt, die die Verpackung für Applikation umhüllt.

23. Verfahren für die Herstellung einer Verpackung zum Aufbringen einer fließfähigen Zusammensetzung, das die beiden nachstehenden Vorgänge umfaßt:
a) Es wird ein Pumpenbalg für manuelle Betätigung (3) so realisiert, daß der besagte Pumpenbalg einen dichten Behälter für die Lagerung der fließfähigen Zusammensetzung bildet, eine Abgabeöffnung für die besagte fließfähige Zusammensetzung aufweist und so angepaßt ist, daß er ergriffen und manuell zusammengekniffen werden kann, damit die fließfähige Zusammensetzung durch die Abgabeöffnung aus dem Behälter befördert werden kann;
b) Es wird ein Applikator (4) realisiert, der eine Innenseite (12) aufweist, die so angepaßt ist, daß sie die Abgabeöffnung des Pumpenbalgs verschließt und somit in direkte Berührung mit der fließfähigen Zusammensetzung gelangt, und eine entgegengesetzte Außenseite (13), die sogenannte Applikationsseite, die so angepaßt ist, daß das Aufbringen der fließfähigen Zusammensetzung auf einer zu behandelnden Fläche möglich wird, wobei der besagte Applikator (4) angepaßt ist, um die Verbreitung der fließfähigen Zusammensetzung bis zur Applikationsseite (13) zuzulassen, wenn der Pumpenbalg (3) zusammengekniffen wird;
**Dadurch gekennzeichnet, daß** bei Vorgang b) der Applikator der besagten Verpackung für Applikation realisiert wird, der aus einem Applikatorkissen aus einem einzigen Teil besteht und mindestens eine Schicht (11) umfaßt, die sogenannte kontinuierliche Filterschicht aus einem geeigneten Material, damit sie für die fließfähige Zusammensetzung (8) durchlässig ist, wenn der Pumpenbalg (3) zusammengekniffen wird, und für die fließfähige Zusammensetzung undurchlässig ist, wenn der Pumpenbalg nicht zusammengekniffen wird.

24. Verfahren nach Anspruch 23, **dadurch gekennzeichnet, daß** bei Vorgang a):
- Eine Platte aus einem bei Raumtemperatur weichen thermoplastischen Material verwendet wird;
- Die besagte thermoplastische Platte auf eine Temperatur ihres plastischen Bereichs erhitzt wird;
- Die erhitzte thermoplastische Platte vermittels eines Stempels und einer Matrix tiefgezogen wird, mit denen auf einer Seite der Platte mindestens eine Austiefung (7) geformt werden kann, die jeweils einen Behälter definiert, und auf der anderen Seite der Platte eine Protuberanz, die die Austiefung(en) aufnimmt und den Pumpenbalg (3) definiert, wobei das gewählte thermoplastische Material und/oder die Erhitzungstemperatur angepaßt ist/sind, damit der somit gebildete Pumpenbalg gegenüber der fließfähigen Zusammensetzung dicht ist.

25. Verfahren nach einem der Ansprüche 23 oder 24, **dadurch gekennzeichnet, daß** bei Vorgang b) eine Platte aus porösem Schaumstoff benutzt wird, die eine Vorderseite aufweist, die für die Ausführung der Innenseite (12) des Applikatorkissens bestimmt ist, und eine entgegengesetzte Vorderseite, die für die Ausführung der Applikationsseite (13) des Applikatorkissens bestimmt ist, damit ein Applikatorkissen (4) realisiert wird, das aus einem einzigen Teil aus porösem Schaumstoff besteht.

26. Verfahren nach Anspruch 25, **dadurch gekennzeichnet, daß**:
- Eine Platte aus synthetischem Schaumstoff mit offenen Zellen verwendet wird, der geeignet ist, eine Durchlässigkeit aufzuweisen, die die Verbreitung der fließfähigen Zusammensetzung zuläßt, und die eine Dicke über derjenigen des auszuführenden Applikatorkissens aufweist;
- Mindestens eine Vorderseite der besagten Schaumstoffplatte vermittels einer Heizplatte erhitzt wird;
- Die Schaumstoffplatte in einer Richtung, der sogenannten Querrichtung, komprimiert wird, die mindestens deutlich orthogonal zu einer ihrer Vorderseiten verläuft, damit die offenen Zellen der besagten Schaumstoffplatte auf einem Teil ihrer Dicke ab der beheizten Vorderseite verkleinert werden, ohne sich zu verstopfen.

27. Verfahren nach Anspruch 26, **dadurch gekennzeichnet, daß** eine Heizplatte benutzt wird, die auf eine geeignete Temperatur erhitzt wird, damit die Schaumstoffplatte auf einem Teil ihrer Dicke ab der erhitzten Vorderseite eine Temperatur in ihrem plastischen Bereich unter dem Schmelzpunkt erreicht, der insbesondere bei zwischen 140 und 240 Grad Celsius liegt.

28. Verfahren nach einem der Ansprüche 26 oder 27, **dadurch gekennzeichnet, daß** die erhitzte Schaumstoffplatte zwischen zwei Verdichtungsplatten komprimiert wird, die mindestens deutlich parallel im Abstand von zwischen 2 und 15 mm verlaufen, die mindestens deutlich der Dicke des auszuführenden Applikatorkissens (4) entspricht.

29. Verfahren nach Anspruch 28, **dadurch gekennzeichnet, daß** mindestens eine der Verdichtungsplatten eine Heizplatte ist, damit die Schritte der Erhitzung begleitend mit der Verdichtung der Schaumstoffplatte eingesetzt werden können.

30. Verfahren für die Herstellung eines verpackten Produkts, **dadurch gekennzeichnet, daß** ein Pumpenbalg (3) und ein Applikatorkissen (4) nach einem beliebigen der vorstehenden Ansprüche 23 bis 29 ausgeführt wird, es wird eine Menge der fließfähigen Zusammensetzung (8) in jede Austiefung gegeben, die Austiefung(en) wird/werden vermittels des Applikatorkissens verschlossen, das durch Wärmeschweißen oder Kleben am Pumpenbalg um die Abgabeöffnung herum befestigt wird.

31. Verfahren nach Anspruch 30, **dadurch gekennzeichnet, daß** die fließfähige Zusammensetzung vermittels einer Spritze in jede Austiefung eingespritzt wird.

32. Verfahren nach einem der vorstehenden Ansprüche 30 oder 31, **dadurch gekennzeichnet, daß**:
- Eine Schaumstoffplatte und eine Platte aus thermoplastischem Material im Großformat verwendet werden;
- Gegebenenfalls wird mindestens eine Vorderseite der Schaumstoffplatte erhitzt, und die besagte Platte wird vermittels von mindestens einer Heizplatte und Verdichtungsplatten komprimiert, die Heizflächen und/oder Verdichtungsflächen aufweisen, die mindestens deutlich gleich oder größer als diejenigen der Vorderseiten der Schaumstoffplatte sind;
- Die thermoplastische Platte wird bis zu einer Temperatur ihres plastischen Bereichs erhitzt, die erhitzte thermoplastische Platte wird vermittels eines Stempels und einer Matrix tiefgezogen, die geeignet sind, damit auf einer Seite der besagten Platte eine Vielzahl von Austiefungen geformt werden kann, die eine Vielzahl von Behältern definieren, und auf der anderen Seite der besagten Platte eine Vielzahl von Protuberanzen, die jeweils mindestens eine Austiefung aufnehmen und eine Vielzahl von Pumpenbälge definieren, die jeweils so angepaßt sind, daß sie ergriffen und zusammengekniffen werden können, damit sie die fließfähige Zusammensetzung durch eine Abgabeöffnung aus dem/den Behälter(n) fördern können;
- In jede der Austiefungen wird eine Menge fließfähige Zusammensetzung eingegeben;
- Die Schaumstoffplatte wird auf der so geformten thermoplastischen Platte angeordnet, so daß die Austiefungen durch die Schaumstoffplatte geschlossen werden;
- Die Schaumstoffplatte und die thermoplastische Platte werden vermittels eines um jede Abgabeöffnung herum vermittels eines beheizbaren Werkzeugs verschweißt, und die beiden Platten werden um jede der somit realisierten Schweißnähte um eine Abgabeöffnung herum ausgestanzt.

33. Verfahren nach Anspruch 32, **dadurch gekennzeichnet, daß** ein beheizbares Werkzeug benutzt wird, das fähig ist, gleichzeitig die Schweißnaht und die Stanzung der Platten um alle Abgabeöffnungen herum auszuführen.

34. Verfahren nach einem beliebigen der vorstehenden Ansprüche 30 bis 33, **dadurch gekennzeichnet, daß** ein Schutzdeckel des Applikatorkissens hergestellt wird, und der Deckel wird abnehmbar auf dem Pumpenbalg und/oder dem Applikatorkissen angehängt, so daß der Deckel die Applikationsseite des besagten Applikatorkissens abdeckt.
